(19) Europäisches Patentamt
European Patent Office
Office européen des brevets

(11) Veröffentlichungsnummer : **0 132 734**
**B1**

(12) # EUROPÄISCHE PATENTSCHRIFT

(45) Veröffentlichungstag der Patentschrift : **25.03.87**

(21) Anmeldenummer : **84108295.1**

(22) Anmeldetag : **14.07.84**

(51) Int. Cl.⁴ : **C 07 C119/042**, C 07 C121/16, C 07 C 87/22

(54) **Neopentylisocyanate und ihre Herstellung.**

(30) Priorität : **26.07.83 DE 3326874**

(43) Veröffentlichungstag der Anmeldung : **13.02.85 Patentblatt 85/07**

(45) Bekanntmachung des Hinweises auf die Patenterteilung : **25.03.87 Patentblatt 87/13**

(84) Benannte Vertragsstaaten : **BE CH DE FR GB IT LI NL**

(56) Entgegenhaltungen :
**EP-A- 0 025 907**
**US-A- 2 499 847**

(73) Patentinhaber : **BAYER AG**
**Konzernverwaltung RP Patentabteilung**
**D-5090 Leverkusen 1 Bayerwerk (DE)**

(72) Erfinder : **Hagemann, Hermann, Dr.**
**Kandinskystrasse 52**
**D-5090 Leverkusen (DE)**

**EP 0 132 734 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue halogenierte Neopentylisocyanate, sowie ein Verfahren zu ihrer Herstellung. Die neuen halogenierten Neopentylisocyanate sind wertvolle Zwischenprodukte für Synthesen in der organischen Chemie, z. B. auf den Gebieten der Farbstoffe, Pharmazeutika, Pflanzenschutzmittel, Kunststoffe und Kautschukhilfsmittel. Besonders geeignet haben sich die neuen Neopentylisocyanate erwiesen zur Herstellung von neuen Carbamidsäureestern, welche als Synergisten in Insektiziden eingesetzt werden können.

Es wurden die neuen halogenierten Neopentylisocyanate der allgemeinen Formel I

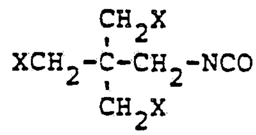

(I)

in welcher X jeweils gleich oder verschieden ist und für Wasserstoff oder Fluor steht, wobei wenigstens ein X Fluor bedeutet,
gefunden.

Weiterhin wurde gefunden, daß man die halogenierten Neopentylisocyanate der allgemeinen Formel I erhält, wenn man

(a) die halogenierten Pivalsäurehalogenide der allgemeinen Formel II

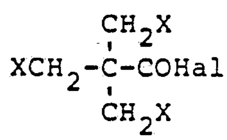

(II)

in welcher
X die oben angegebene Bedeutung hat und
Hal für Fluor oder Chlor steht
mit Ammoniak zu den halogenierten Pivalsäureamiden der allgemeinen Formel III

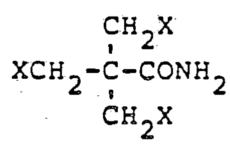

(III)

in welcher X die oben angegebene Bedeutung hat, umsetzt und

(b) die gegebenenfalls isolierten Verbindungen der allgemeinen Formel III mit den üblichen Dehydratisierungsmitteln in die halogenierten Pivalsäurenitrile der allgemeinen Formel IV

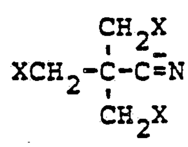

(IV)

in welcher X die oben angegebene Bedeutung hat, überführt und

(c) die gegebenfalls isolierten Verbindungen der allgemeinen Formel IV durch katalytische Hydrierung in die halogenierten Neopentylamine der allgemeinen Formel V

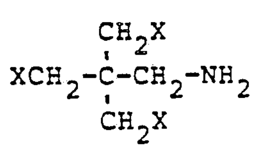

(V)

in welcher X die oben angegebene Bedeutung hat, umwandelt und diese gegebenenfalls mit Chlorwasserstoff in die Hydrochloride umsetzt und

(d) die gegebenenfalls isolierten Verbindungen der Formel V oder deren Hydrochloride mit Phosgen ($COCl_2$) in die halogenierten Neopentylisocyanate der allgemeinen Formel (I) überführt und diese nach

üblichen Methoden isoliert und gegebenenfalls reinigt.

Die in den einzelnen Verfahrensstufen (a) bis (c) erhaltenen Verbindungen der allgemeinen Formeln I bis V können entweder nach üblichen Methoden isoliert oder ohne Isolierung direkt in der nächsten Stufe weiterverarbeitet werden.

Die als Ausgangsstoffe einzusetzenden halogenierten (insbesondere chlorierten) Pivalsäurehalogenide der allgemeinen Formel II sind bekannt und/oder nach allgemein üblichen Verfahern und Methoden erhältlich. Die Pivalsäurehalogenide der allgemeinen Formel II, in welchen Hal Fluor bedeutet, werden erhalten, wenn man die entsprechenden chlorierten Pivalsäurechloride mit einem Alkalifluorid bei erhöhter Temperatur, z. B. bei 200 °C, in einem organischen Lösungsmittel wie Tetramethylensulfon umsetzt, wobei bezogen auf die Zahl der durch Fluor zu ersetzenden Chloratome (Chloratome der Methylgruppen und Chloratom des Säurechloridteiles) die jeweils entsprechende äquimolare Menge Alkalifluorid oder ein geringer Überschuß eingesetzt wird.

Die Reaktionsstufen (a) bis (d) können nach den üblichen Methoden der organischen Chemie durchgeführt werden.

Die Umsetzungen werden im allgemeinen in Gegenwart eines organischen Lösungsmittels, welches sich in der jeweiligen Reaktionsstufe gegenüber den Reaktanden inert verhält, durchgeführt.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol, und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon und Hexamethylphosphorsäuretriamid sowie Mischungen dieser Lösungsmittel.

In der Reaktionsstufe (a) wird Ammoniak gasförmig in eine Lösung der Verbindung der allgemeinen Formel II eingeleitet oder es wird eine wäßrige Ammoniak-Lösung (z. B. 25 Gew.-%) eingesetzt. Das Ammoniak kann in äquimolarer Menge oder aber auch in einem Überschuß bis zu etwa der 3-fachen molaren Menge eingesetzt werden. Die Reaktionstemperaturen liegen beim Verfahrensschritt (a) zwischen — 20 bis + 300 °C, vorzugsweise zwischen 0 und 80 °C. Das halogenierte Pivalsäureamid der allgemeinen Formel III kann nach üblichen Methoden isoliert und gegebenenfalls gereinigt werden (z. B. durch Absaugen oder Entfernen der Lösungsmittel durch Destillation).

In der Reaktionsstufe (b) werden die halogenierten Pivalsäureamide der allgemeinen Formel III ohne Lösungsmittel oder in Gegenwart eines vorzugsweise höher-siedenden Lösungsmittels (z. B. Xylol, o-Dichlorbenzol oder Sulfolan) mit einem üblichen wasserabspaltenden Reagenz, wie $P_2O_5$, $Al_2O_3$, $SOCl_2$ oder $COCl_2$ umgesetzt, wobei diese dehydratisierenden Mittel in der theoretisch erforderlichen Menge oder aber im Überschuß (z. B. bis zur doppelten erforderlichen Menge) verwendet werden. Die Umsetzung (b) kann unter Normaldruck, aber auch unter vermindertem Druck (z. B. 10 bis 500 mbar) bei erhöhten Temperaturen, vorzugsweise zwischen 80 und 300 °C, insbesondere zwischen 120 und 180 °C vorgenommen werden. Die erhaltenen halogenierten Pivalsäurenitrile der allgemeinen Formel IV werden gewünschtenfalls nach den allgemeinen üblichen Methoden (z. B. durch Destillation) isoliert und gegebenenfalls gereinigt.

In der Reaktionsstufe (c) werden die halogenierten Pivalsäurenitrile der allgemeinen Formel IV mit den üblichen Hydrierungskatalysatoren (wie Raney-Nickel, Palladium oder Platin) unter den üblichen Bedingungen einer Hydrierung in die entsprechenden Amine übergeführt. Zweckmäßigerweise wird die Hydrierung unter einem Wasserstoffüberdruck (z. B. 10 bis 120, vorzugsweise 40 bis 100 bar) und bei Temperaturen von 20 bis 60, vorzugsweise 30 bis 50 °C vorgenommen. Bevorzugt werden als Lösungsmittel niedere Alkylalkohole, wie Methanol oder Ethanol eingesetzt. Zur Aufarbeitung in üblicher Weise wird der Katalysator abfiltriert und das Lösungsmittel durch Destillation entfernt. Falls das Amin-Hydrochlorid hergestellt werden soll, wird zweckmäßigerweise die alkoholische Amin-Lösung mit Chlorwasserstoff (z. B. durch Einleiten von Chlorwasserstoffgas oder Hinzufügen einer alkoholischen Chlorwasserstoff-Lösung) sauer gestellt. Das Aminhydrochlorid wird abgesaugt oder nach Abdestillieren des Lösungsmittels erhalten.

In der Reaktionsstufe (d) wird die Phosgenierung zweckmäßigerweise durch Erwämen der halogenierten Neopentylamine der allgemeinen Formel V oder deren Hydrochloride in einer gesättigten Phosgenlösung, z. B. in Chlorbenzol, unter zusätzlichem Einleiten von Phosgen durchgeführt. Die Umsetzung ist abgeschlossen, wenn kein Chlorwasserstoffgas mehr entweicht. Der restliche Chlorwasserstoff kann durch Einleiten von Stickstoff aus dem Reaktionsgemisch getrieben werden. Die Umsetzung wird bei Temperaturen zwischen 0 und 150, vorzugsweise zwischen 15 und 135 °C durchgeführt. Die Isolierung und Reinigung der halogenierten Neopentylisocyanate der allgemeinen Formel I erfolgt nach den üblichen Methoden, z. B. durch Destillation.

Wie bereits oben erwähnt, können die erfindungsgemäßen Verbindungen als Zwischenprodukte für Synthesen in der organischen Chemie auf den verschiedensten Gebieten eingesetzt werden. In besonders vorteilhafter Weise können die halogenierten Neopentylisocyanate der allgemeinen Formel I mit Propargylalkohol in Carbamidsäureester übergeführt werden, welche in überraschender Weise sehr hohe synergistische Wirksamkeiten zusammen mit arthropodiziden Wirkstoffen (z. B. dem insektiziden

Carbamat Propoxur) entfalten und somit in insektiziden Schädlingsbekämpfungsmitteln verwendet werden können. In EP-A-0 025 907 wird sehr allgemeinen die Verwendbarkeit von halogenierten tertiären Alkylisocyanaten zur Herstellung von u. a. auch Schädlingsbekämpfungsmitteln vorgeschlagen. Es war jedoch überraschend, daß die neuen Verbindungen der Formel (I) aufgrund ihrer speziellen Struktur zu synergistisch wirkenden Verbindungen führen, welche in Schädlingsbekämpfungsmitteln verwendet werden können.

Zur Herstellung der synergistisch wirkenden Carbamidsäureester werden die halogenierten Neopentylisocyanate der allgemeinen Formel I z. B. mit der äquimolaren Menge Propargylalkohol in Toluol bei Raumtemperatur (ca. 20 °C) unter Zusatz geringer Mengen Diazabicyclooctan umgesetzt. Nach üblicher Aufarbeitung (Entfernen der Lösungsmittel) erhält man die synergistisch wirkenden halogenierten N-(2,2-Dimethyl-3-propyl)carbamidsäure-0-2-propinylester.

Die Anwendung dieser Verbindungen in Mischung mit insektiziden Wirkstoffen, wie dem Carbamat Propoxur, erfolgt in üblicher Weise, z. B. in Form von Aerosolen oder Spritzpulver. Diese Zubereitungen eignen sich in hervorragender Weise zur Bekämpfung von Insekten, z. B. zur Stubenfliegen-Bekämpfung.

Die Herstellung der erfindungsgemäßen Verbindungen soll anhand der folgenden Beispiele erläutert werden :

Beispiel 1

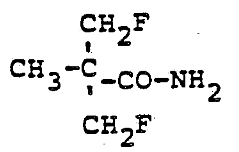 aus 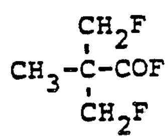

Es werden 700 g 25 %ige (Gew.-%) wäßrige Ammoniak-Lösung und 500 g Eis vorgelegt und 515 g (3,7 mol) Difluorpivalsäurefluorid (Bisfluormethylpropionsäurefluorid) gelöst in 250 ml $CH_2Cl_2$ zugetropft.

Man läßt bei ca. 10 °C 15 Min. nachrühren, saugt den Niederschlag ab, wäscht mit etwas kaltem Wasser und trocknet bei 70 °C unter vermindertem Druck.

Ausbeute : 451 g (89 % d. Th) Bisfluormethylpropionsäureamid ; Fp. : 100-101 °C.

Beispiel 2

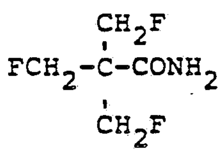 aus 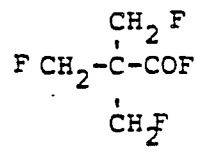

In analoger Weise wie in Beispiel 1 beschrieben, jedoch mit Xylol als Lösungsmittel und bei einer Reaktionstemperatur von 40-50 °C wird aus Trifluormethylessigsäurefluorid (Trifluorpivalsäurefluorid) in einer Ausbeute > 95 % der Theorie Trisfluormethylessigsäureamid erhalten. Fp. : 114-115 °C.

Beispiel 3

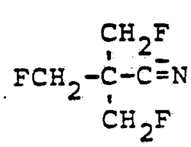 aus 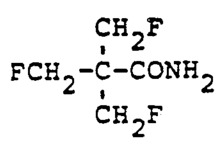

Es werden 250 g (1,6 mol) Trisfluormethyl-essigsäureamid und 284 g (2 mol) $P_2O_5$ gut durchmischt und bei ca. 70 mbar und 160-200 °C Außentemperatur erhitzt, wobei das Nitril abdestilliert. Der Versuch wird in dem gleichen Reaktionsgefäß mit dem Rückstand wiederholt und man erhält insgesamt 434 g (89 % der Theorie) Trisfluormethylacetonitril, Kp. 90 °C/58 mbar ; Fp. : 70-75 °C.

Beispiel 4

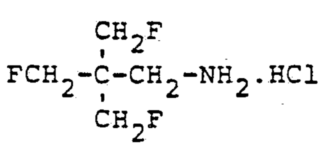 aus 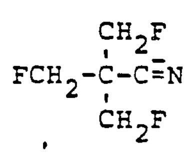

30 g (4,218 mol) Trisfluormethylessigsäurenitril werden in 180 ml Methanol gelöst und in Gegenwart

von 10 g Raney-Nickel (Ni : Fe 85 : 15) bei 80-90 bar $H_2$ und einer Temperatur von 38-40 °C hydriert. Es wird filtriert und das Filtrat im Wasserstrahlvakuum bis zur Trockne von Methanol befreit. Die methanolische Lösung wird durch Einleiten von HCl-Gas sauer gestellt, worauf das Aminhydrochlorid ausfällt. man saugt ab und erhält 36 g (93 % der Theorie) Trifluorneopentylaminhydrochlorid.

Beispiel 5

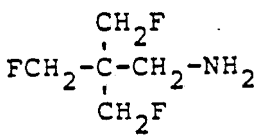

aus

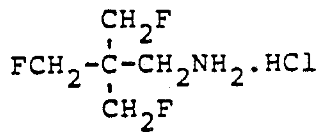

106,5 g (0,5 Mol) Trifluorneopentylaminhydrochlorid werden unter Eiskühlung in eine Lösung von 200 g 50 %igen KOH in 40 ml $H_2O$ eingetragen, das freie Amin mit 250 ml Ether ausgeschüttelt, mit $MgSO_4$ getrocknet, eingeengt und der feste Rückstand unter vermindertem Druck 1 Std. bei 20 °C getrocknet. Ausbeute an freiem Amin : 6,6 g (78 % der Theorie) Fp. : 66-67 °C, Kp. : 74 °C/52 mbar.

Die Herstellung der fluorierten Pivaloylfluoride der allgemeinen Formel II soll anhand der folgenden Beispiele erläutert werden :

Beispiel 1A

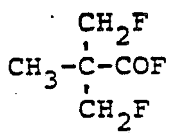

aus

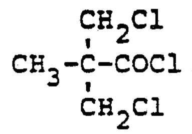

Es werden 947,5 g (5 mol) Dichlorpivaloylchlorid, 2 kg Tetramethylensulfon und 1,16 kg (20 mol) Kaliumfluorid bei einem Anfangsdruck von 2 bar $N_2$ für 5 Std. bei 230 °C in einem VA-Rührautoklaven erhitzt, abgekühlt, entspannt und unter leicht vermindertem Druck destilliert. Man erhält 576 g (79,2 % d. Th.) Difluorpivaloylfluorid (Kp. 110-120 mbar 52-56 °C). Im 3 kg-Maßstab in einem Schaufelreaktor erhält man Ausbeuten zwischen 85 und 90 % d. Th.

Beispiel 2A

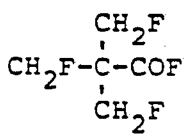

aus

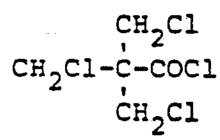

Es werden 900 g (4 mol) Trischlormethylessigsäurechlorid (Trichlorpivaloylchlorid), 1,16 kg (20 mol) Kaliumfluorid und 2 250 ml Tetramethylensulfon unter Normaldruck 5 Std. bei 200 °C gerührt, abgekühlt, mit 1 l Xylol versetzt und bis zum Siedepunkt des Tetramethylensulfons andestilliert. Die Xylollösung enthält das gesamte Trisfluormethylessigsäurefluorid (80 % der Theorie), das wegen seines Schmelzpunkts von 50-52 °C, des Siedepunkts von 48 °C/22 mbar und der hohen Reaktivität des Säurefluorids im allgemeinen in der Lösung direkt weiter umgesetzt wird.

Beispiel 3A

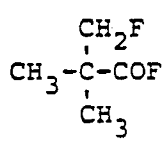

aus

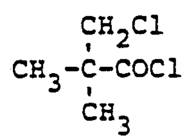

5,8 kg (100 mol) Kaliumfluorid und 7,7 kg Tetramethylensulfon werden in einem 20 l-Schaufelreaktor mit Destillationsaufbau vorgelegt und bei ca. 20 mbar unter Abnahme von 10 % des eingesetzten Lösungsmittels andestilliert. Es wird mit $N_2$ belüftet, die Innentemperatur von 150 °C auf 125-130 °C fallengelassen, 6,2 kg (40 ml) Chlorpivaloylchlorid eingesaugt, die Apparatur mit $N_2$ gespült und druckdicht verschlossen. Nach Aufpressen von 3 bar $N_2$ wird 1 Std. auf 150 °C und 12 Std. auf 230 °C erwärmt, auf 80 °C abgekühlt und bei 100 mbar destilliert.

Man erhält 3,256 kg (68 % der Theorie) Fluorpivaloylfluorid (Sdp. 40-41 °C/100 mbar) und 1,4 kg (26 %) Chlorpivaloylfluorid (Sdp. 65 °C/100 mbar) als Nebenprodukt. Das Ergebnis entspricht einer

Selektivität von 92 % bei 74 % Umsatz.

Die Herstellung der synergistisch wirksamen Carbamidsäureester soll anhand der folgenden Herstellungsbeispiele erläutert werden :

Beispiel 1B

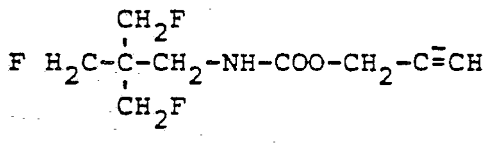

N-(2,2-Bisfluormethyl-3-fluorpropyl)-carbamidsäure-0-2-propinylester

16,7 g 2,2-Bisfluormethyl-3-fluorpropyl-isocyanat (29 %ige Lösung in Toluol) werden in 100 ml Toluol gelöst und 10 mg Diazabicyclooctan (Dabco) zugesetzt. Zu diesem Reaktionsgemisch tropft man bei Raumtemperatur (ca. 20 °C) 5,6 g 2-Propinyl-alkohol.

Nach vierstündigem Erhitzen des Gemisches zum Sieden wird das Reaktionsgemisch abgekühlt und mit Wasser gewaschen. Nach Trocknen über Natriumsulfat wird das Lösungsmittel abdestilliert und das zurückbleibende Öl im Hochvakuum vom restlichen Lösungsmittel befreit. Man erhält 22 g N-(2,2-Difluormethyl-3-fluorpropyl)-carbamidsäure-0-2-propinylester in Form eines farblosen Öls (80 %ig der Theorie Ausbeute), $n_D^{20}$ : 1,450 5.

Die Verbindungen der folgenden Beispiele werden in analoger Weise erhalten :

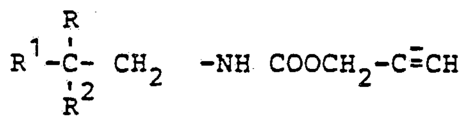

| Beispiel Nr. | R | $R^1$ | $R^2$ | Brechungsindex $n_D^{20}$ °C |
|---|---|---|---|---|
| 2 B | $CH_2F$ | $CH_3$ | $CH_2F$ | 1,4505 |
| 3 B | $CH_3$ | $CH_2F$ | $CH_3$ | 1,4580 |

Die überraschende synergistische Wirksamkeit der Carbamidsäureester ist aus den folgenden Versuchsergebnissen ersichtlich :

Angewendete Testmethode :

$LT_{100}$-Test

Testtiere : Musca domestica ♀♀, Stamm Weymanns (resistent)

Lösungsmittel : Aceton

Von den Wirkstoffen, Synergisten und Gemischen aus Wirkstoffen und Synergisten werden Lösungen hergestellt und 2,5 ml davon in Petrischalen auf Filterpapier von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Tiere wird bis zu 6 Stunden fortlaufend kontrolliert. Es wird diejenige Zeit ermittelt, die für eine 100 %ige knock down-Wirkung erforderlich ist. Wird die $LT_{100}$ nach 6 Stunden nicht erreicht, wird der % Satz der knock down gegangenen Testtiere festgestellt.

Wirkstoffe, Synergisten, die Konzentrationen der Wirkstoffe, Synergisten und Gemische sowie ihre Wirkungen gehen aus der nachfolgenden Tabelle hervor.

Als insektizider Wirkstoff wurde Propoxur der Formel A

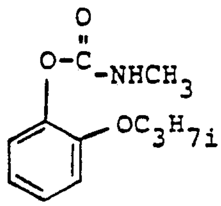

(A)

eingesetzt.

Testergebnisse :

| Wirkstoff | | Synergist | Konzentrationen in % Wirkstoff | | Synergist | $LT_{100}$ in Minuten oder bei 360' in % |
|---|---|---|---|---|---|---|
| (A) | | - | 1,0 | | - | 360' = 60 % |
| - | | 1B | - | | 1,0 | 360' = 35 % |
| - | | 2B | - | | 0,2 | 360' = 90 % |
| - | | 3B | - | | 0,2 | 360' |
| (A) | + | 1B | 0,04 | + | 0,04 | 90' |
| (A) | + | 2B | 0,04 | + | 0,04 | 90' |
| (A) | + | 3B | 0,04 | + | 0,04 | 75' |

Die Anwendung der insektiziden Mittel erfolgt in üblicher Weise z. B. in Form von Aerosolen, Spritzpulver usw.

**Patentansprüche**

1. Halogenierte Neopentylisocyanate der allgemeinen Formel I

$$\begin{array}{c} CH_2X \\ XCH_2-C-CH_2-NCO \\ CH_2X \end{array} \qquad (I)$$

in welcher X jeweils gleich oder verschieden ist und für Wasserstoff oder Fluor steht, wobei wenigstens ein X Fluor bedeutet.

2. Verfahren zur Herstellung von halogenierten Neopentylisocyanaten der allgemeinen Formel I

$$\begin{array}{c} CH_2X \\ XCH_2-C-CH_2-NCO \\ CH_2X \end{array} \qquad (I)$$

in welcher X jeweils gleich oder verschieden ist und für Wasserstoff oder Fluor steht, wobei wenigstens ein X Fluor bedeutet,
dadurch gekennzeichnet, daß man

(a) die halogenierten Pivalsäurehalogenide der allgemeinen Formel II

$$\begin{array}{c} CH_2X \\ XCH_2-C-CO\ Hal \\ CH_2X \end{array} \qquad (II)$$

in welcher
X die oben angegebene Bedeutung hat und
Hal für Fluor oder Chlor steht
mit Ammoniak zu den halogenierten Pivalsäureamiden der allgemeinen Formel III

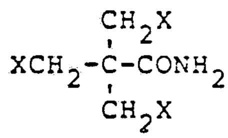
(III)

in welcher X die oben angegebene Bedeutung hat, umsetzt und

(b) die gegebenenfalls isolierten Verbindungen der allgemeinen Formel III mit den üblichen Dehydratisierungsmitteln in die halogenierten Pivalsäurenitrile der allgemeinen Formel IV

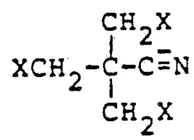
(IV)

in welcher X die oben angegebene Bedeutung hat, überführt und

(c) die gegebenenfalls isolierten Verbindungen der allgemeinen Formel IV durch katalytische Hydrierung in die halogenierten Neopentylamine der allgemeinen Formel V

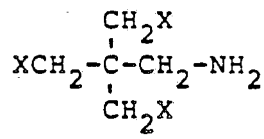
(V)

in welcher X die oben angegebene Bedeutung hat, umwandelt und diese gegebenenfalls mit Chlorwasserstoff in die Hydrochloride umsetzt, und

(d) die gegebenenfalls isolierten Verbindungen der Formel V oder deren Hydrochloride mit Phosgen ($COCl_2$) in die halogenierten Neopentylisocyanate der allgemeinen Formel (I) überführt und diese nach üblichen Methoden isoliert und gegebenenfalls reinigt.

**Claims**

1. Halogenated neopentyl isocyanates of the general formula I

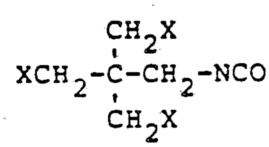
(I)

in which X is in each case identical or different and represents hydrogen or fluorine, at least one X denoting fluorine.

2. Process for the preparation of halogenated neopentyl isocyanates of the general formula I

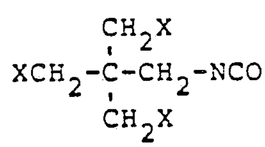
(I)

in which X is in each case identical or different and represents hydrogen or fluorine, at least one X denoting fluorine,
characterised in that

(a) the halogenated pivalic acid halides of the general formula II

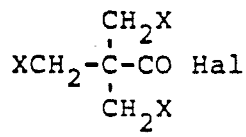 (II)

in which
X has the abovementioned meaning and
Hal represents fluorine or chlorine,
are reacted with ammonia to form the halogenated pivalic acid amides of the general formula III

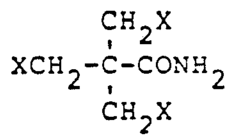 (III)

in which X has the abovementioned meaning, and
(b) the optionally isolated compounds of the general formula III are converted with the customary dehydrating agents into the halogenated pivalic acid nitriles of the general formula IV

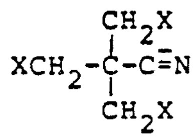 (IV)

in which X has the abovementioned meaning, and
(c) the optionally isolated compounds of the general formula IV are converted by catalytic hydrogenation into the halogenated neopentyl amines of the general formula V

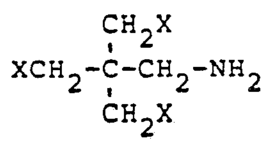 (V)

in which X has the abovementioned meaning, and these are optionally converted into the hydrochlorides with hydrogen chloride, and
(d) the optionally isolated compounds of the formula V or their hydrochlorides are converted with phosgene ($COCl_2$) into the halogenated neopentyl isocyanates of the general formula (I) and these are isolated by customary methods and optionally purified.

**Revendications**

1. Isocyanates de néopentyle halogénés de formule générale I

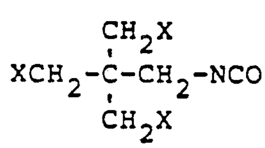 (I)

dans laquelle les symboles X, ayant des significations identiques ou différentes, représentent l'hydrogène ou le fluor, l'un au moins de ces symboles représentant le fluor.
2. Procédé de préparation des isocyanates de néopentyle halogénés de formule I

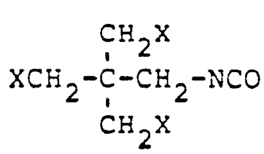 (I)

dans laquelle les symboles X, ayant des significations identiques ou différentes, représentent l'hydrogène ou le fluor, l'un au moins de ces symboles représentant le fluor,
caractérisé en ce que :

(a) on fait réagir les halogénures d'acides pivaliques halogénés de formule générale II

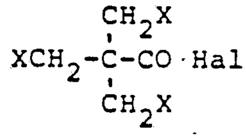

(II)

dans laquelle

X a les significations indiquées ci-dessus, et

Hal représente le fluor ou le chlore,

avec l'ammoniac, ce qui donne les amides d'acides pivaliques halogénés de formule III

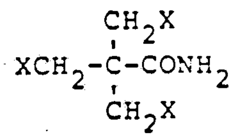

(III)

dans laquelle X a les significations indiquées ci-dessus, et

(b) on convertit les composés de formule générale III, éventuellement après isolement, à l'aide des agents déshydratants usuels, en les nitriles d'acides pivaliques halogénés de formule générale IV

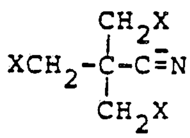

(IV)

dans laquelle X a les significations indiquées ci-dessus, et

(c) on convertit les composés de formule générale IV, éventuellement après isolement, par hydrogénation catalytique, en les néopentylamines halogénées de formule générale V

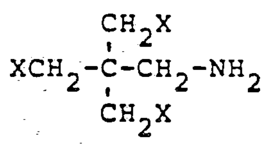

(V)

dans laquelle X a les significations indiquées ci-dessus, et, le cas échéant, on convertit ces amines en les chlorhydrates à l'aide de chlorure d'hydrogène, et

(d) on convertit les composés de formule V ou leurs chlorhydrates, éventuellement après isolement, par le phosgène $COCl_2$, en les isocyanates de néopentyle halogénés de formule générale I qu'on isole et, le cas échéant, qu'on purifie par des techniques usuelles.